# EUROPEAN PATENT APPLICATION

(11) **EP 1 790 313 A2**
(43) Date of publication of application: **30.05.2007**
(21) Application number: 06256074.3
(22) Date of filing: 28.11.2006
(51) Int. Cl.: A61F 2/04

(54) **Internally placed gastric restriction device**

(30) Priority: 29.11.2005 US 164575
(71) Applicant: ETHICON ENDO-SURGERY, INC., Cincinnati, Ohio 45242 (US)
(72) Inventor: Ortiz, Mark S., Milford, OH 45150 (US); Byrum, Randal T., Kings Mill, OH 45034 (US)
(74) Representative: Tunstall, Christopher Stephen

(57) **Abstract**

Various devices for reducing the size of a passageway, such as, in an exemplary embodiment, a gastrointestinal passageway are provided. The device generally includes a cylindrical member, serving as a restriction element, having proximal and distal ends, at least one of which can be adapted to couple to a wall of the gastrointestinal passageway such that the size of the passageway decreases. In use, the restriction element is selectively configurable in a first, inactivated position and a second, activated position, such that a diameter of the restriction element is larger in the activated position than in the inactivated position, and the size of the passageway can be controlled. A method of suppressing appetite using such a device is also provided.

## Description

### FIELD OF THE INVENTION

The present invention relates to devices and methods for bariatric surgery, and in particular, to internally placed gastric restriction devices and methods.

### BACKGROUND OF THE INVENTION

One treatment for morbid obesity is bariatric surgery which involves alteration of a patient's digestive tract to encourage weight loss and to help maintain a normal weight. A common type of bariatric surgery is gastric bypass surgery, which aims to decrease the size of a patient's stomach by dividing it into upper and lower pouches using staples and/or stitches. The jejumum (the middle section of the small intestine) is also divided into two parts. One part of the jejunum (called the "Roux limb") is brought up behind the colon and lower stomach pouch, and joined or "anastamosed" to the upper stomach pouch. The remaining end of the jejunum is attached to the side of the Roux limb. As a result, a new digestive pathway is created, through which food travels down the esophagus, into the upper stomach pouch, and through the anastamosis into the Roux limb. Digestive juices from the stomach, the liver, and the pancreas travel through the lower stomach pouch, down the duodenum and jejunum, and into the Roux limb where the two parts of the jejunum are attached and further digestion takes place.

While effective, gastric bypass surgery is not without complications, and such complications include leaks at the junction of stomach and small intestine and the development of a stricture in the stoma (the junction between the upper stomach pouch and the Roux limb) as a result of the formation of scar tissue. To remedy these complications, a patient must undergo further surgery which can often lead to additional complications and a more difficult recovery period.

Another treatment for morbid obesity is gastric band surgery, where an adjustable ring or band is placed completely around the top end (fundus) of the stomach and constricted to create an hourglass effect to limit a patient's food intake. The diameter of the band can subsequently be adjusted depending on the needs of the patient. One example of an adjustable band is a band that includes an inflatable balloon attached thereto. This inflatable balloon is connected through tubing to a port placed under the patient's skin. Using needles inserted into the port, saline or another fluid is passed through the tubing to inflate or deflate the balloon to decrease or increase the size of the band's diameter, thus controlling the food intake of the patient. While this inflatable band eliminates the need for additional surgery to adjust the band, problems can arise through the use of the port and the needles to insert the saline. For example, repeated adjustment can cause scarring on the skin around the port. Further, the use of a fluid-filled balloon can pose a potential risk of puncture or over-inflation.

Accordingly, there remains a need for improved bariatric surgery devices and methods, and in particular for improved gastric restriction devices and methods.

### SUMMARY OF THE INVENTION

The present invention provides various devices and methods for controlling a size of the gastrointestinal passageway. The devices and methods described herein can be used to suppress one's appetite and thus to reduce a patient's weight. For example, the device can be used to restrict the size of a gastrointestinal passageway such as the stomach. In one aspect, the stomach volume can be reduced to control appetite and result in weight loss.

In one aspect, a device includes a cylindrical member, acting as a restriction element, having proximal and distal ends, with at least one of the proximal and distal ends being adapted to couple to a wall of the gastrointestinal passageway such that the size of the passageway is decreased. The member is selectively configurable in a first inactivated position and a second activated position, such that a diameter of the member is larger in the activated position than in the inactivated position. Accordingly, the size of the gastrointestinal passageway can be reduced, which can decrease the volume of the stomach and lead to a sense of satiety with less food intake.

The restriction element can have a variety of configurations. In one embodiment, the restriction element can be a wire mesh stent having proximal and distal ends that, when moved from an inactivated position to an activated position, radially expands and axially contracts. The stent also includes a plurality of circumferentially disposed wire ends that are adapted to be disposed radially outwardly when the stent is in the activated position. By way of non-limiting example, the diameter of the stent can increase about 2.5 times when the stent is moved from the inactivated position to the activated position.

In another embodiment, the restriction element can include an upper ring and a lower ring having a seal located therebetween. The seal extends inwardly from the upper and lower rings and defines an opening which can have a selectively controllable diameter. The upper ring can optionally be adapted to rotate relative to the lower ring to control the diameter of the opening. The device can also include a variety of other features, such as features that allow it to couple to the tissue of the passageway. For example, a distal portion of the outer surface of the lower ring can include a wire mesh band that is attached thereto, and/or an attachment element that is adapted to receive a fastener that can couple the lower ring to the wall of the gastrointestinal passageway.

In another aspect, a system for controlling the size of a gastrointestinal passageway is provided that includes a tubular restriction element adapted to couple to a wall of the gastrointestinal passageway such that the size of the passageway is decreased. The restriction element has a diameter that is adjustable when the restriction element is moved from an inactivated position to an activated position. The system also includes an activation device that is adapted to manipulate the restriction element such that it moves from the inactivated position to the activated position. The activation device can be a delivery device, or components of the delivery device, or it can be an implement separate from the delivery device.

In one embodiment, the restriction element can be a wire mesh stent that is adapted to radially expand and axially contract when moved from the inactivated position to the activated position. The restriction element can also include proximal and distal ends with plurality of circumferentially disposed wire ends formed thereon that are adapted to engage tissue upon movement of the stent from the inactivated position to the activated position. The activation device can include proximal and distal portions that are respectively adapted to engage the proximal and distal ends of the stent in the inactivated position as well as selectively release the proximal and distal ends of the stent to effect activation thereof. In another embodiment, the restriction element can be a cylindrical housing or adjustable ring that has a seal coupled between upper and lower rings and extending inwardly therefrom to define an opening. The diameter of the opening can be selectively controlled by relative rotation of the upper and lower rings.

In another aspect, methods for appetite suppression are also disclosed. One exemplary method includes inserting a restriction element to a site within the gastrointestinal passageway in an inactivated position, and coupling at least a portion of the restriction element to at least a portion of a wall of the gastrointestinal passageway at the site. The method further includes activating the restriction element such that the restriction element changes diameter thereby effecting a change in diameter of the gastrointestinal passageway.

The restriction element can be inserted into the passageway in a variety of ways, for example by using a delivery device, such as a grasper, or by attaching a guide wire thereto and using the guide wire to facilitate insertion of the restriction element within the passageway. The restriction element can be coupled to the walls of the passageway by a variety of techniques, and in one embodiment, a portion of the gastrointestinal passageway can be suctioned such that it is pulled inwardly towards the outer circumference of the restriction element to adhere to an outer circumference of the restriction element and to thereby reduce the volume of the passageway. Further, when the restriction element is released from the delivery device, the radially expandable wire ends formed on the proximal and distal ends thereof engage the wall of the gastrointestinal passageway and maintain the passageway in a reduced volume condition. Alternatively, a fastener can be inserted between an attachment element formed on a portion of the restriction element and the wall of the passageway. The restriction element can also be activated using a variety of techniques and devices. In one embodiment, the restriction element can be released from a delivery device or an activation element such that it radially expands and axially contracts.

### BRIEF DESCRIPTION OF THE DRAWINGS

The invention will be more fully understood from the following detailed description taken in conjunction with the accompanying drawings, in which:

FIG. 1 A is a side perspective view of one exemplary embodiment of an internally placed gastric restriction device in an inactivated position;

FIG. 1B is a side perspective view of the gastric restriction device of FIG. 1A in an activated position;

FIG. 2A is a top perspective view of another exemplary embodiment of an internally placed gastric restriction device in one activated position;

FIG. 2B is a top perspective view of the gastric restriction device in another activated position;

FIG. 3A is a side perspective view of an exemplary system for inserting a gastric restriction device that includes the gastric restriction device of FIGS. 1A-1B and an activation device in the inactivated position;

FIG. 3B is an illustration of the system of FIG. 3A upon insertion into the stomach;

FIG. 3C is a side perspective view of the system of FIG. 3A in the activated position; and

FIG. 3D is an illustration of a stomach following the placement of any of the gastric restriction devices disclosed herein.

### DETAILED DESCRIPTION OF THE INVENTION

Certain exemplary embodiments will now be described to provide an overall understanding of the principles of the structure, function, manufacture, and use of the devices and methods disclosed herein. One or more examples of these embodiments are illustrated in the accompanying drawings. Those of ordinary skill in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

The present invention provides various devices and methods for reducing the size of a passageway, such as a gastrointestinal passageway (e.g., the stomach). The device generally includes a cylindrical member, serving as a restriction element, having proximal and distal ends, at least one of which can be adapted to couple to a wall of the gastrointestinal passageway such that the size of the passageway decreases. While the restriction element can have a variety of configurations, in an exemplary embodiment the restriction element can be a stent or a cylindrical member, both of which will be discussed in more detail below. In use, the restriction element is selectively configurable in a first, inactivated position which is suited for endoscopic delivery to an intended site and a second activated position which is achieved upon deployment at the intended site. The diameter of the restriction element is larger in the activated position than in the inactivated position, but it is still of a size that enables it to restrict the size of a passageway within the body. While the exemplary device, system, and method are discussed with respect to use in bariatric surgery, and in particular, for reducing the volume of the stomach, one skilled in the art will appreciate that the device can be used in a variety of other applications.

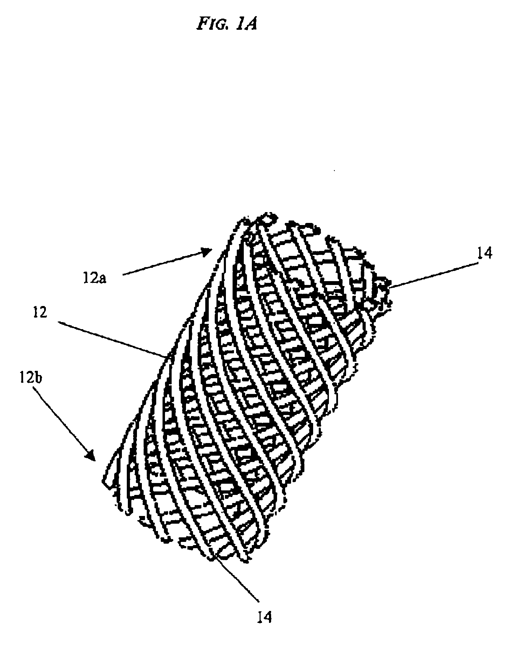

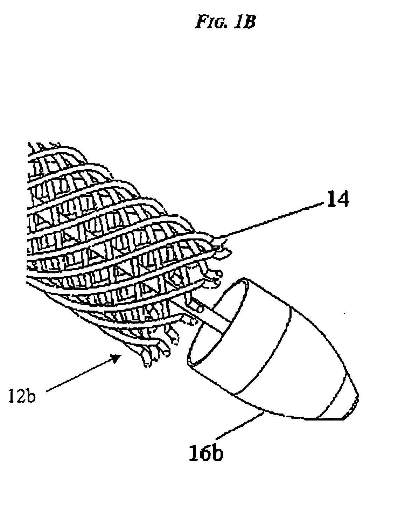

In one embodiment, as shown in FIGS. 1A-1B, the restriction element can be a stent 12 having proximal and distal ends 12a, 12b with plurality of circumferentially disposed wire ends 14 formed thereon. Upon movement of the stent 12 from an inactivated position (FIG. 1A) to an activated position (FIG. 1 B), the wire ends 14 can move radially outwardly to enable the stent 12 to engage the wall of the passageway. Further, reconfiguring the stent 12 from the inactivated position to the activated position causes the stent to axially contract and radially expand within the passageway. Although the stent has a diameter in the activated position that is larger than the diameter in the inactivated (delivery) position, it is still smaller than the natural opening or size of the passageway and it is thus effective in appetite control and weight loss.

In particular, FIG. 1A shows the stent 12 in an inactivated position, where it is radially contracted and axially expanded and thus suitably sized for endoscopic (e.g., transoral) delivery. The inactivated stent 12 can have any diameter that allows for endoscopic delivery of the device and insertion into the passageway, however in an exemplary embodiment, where the passageway is the gastrointestinal passageway, the diameter of the stent 12 in the inactivated position can be in the range of about 5-15 mm, and more preferably about 10 mm. The length of the inactivated stent 12 can be adapted such that upon activation it will contract to a desired length. In an exemplary embodiment the length of the inactivated stent can be in the range of about 50mm or less.

Once the stent 12 is positioned at the desired tissue site, the stent 12 can reconfigure from the inactivated position to the activated position to effect coupling with the side of the passageway as well as to effect a change in the diameter of the passageway. In particular, as shown in FIG. 1 B and as discussed below, the stent 12 can be freed from a delivery device (16 in FIG. 3C), such that it moves from the inactivated position to the activated position. That is, the stent 12 radially expands and axially contracts upon being released from the delivery device such that the diameter of the stent 12 increases (e.g., by about 2.5 times) to effect a change in the size of the passageway. By way of non-limiting example, when the diameter of the stent 12 in the inactivated position is about 5-15 mm, the diameter of the stent 12 in the activated position can be in the range of about 20-30 mm, and more preferably when the diameter of the stent 12 in the inactivated position is about 10 mm, the diameter of the stent 12 in the activated position can be about 25 mm. Additionally, as a result of activation of the stent 12 (i.e., by radial expansion and axial contraction), the wire ends 14 formed on the proximal and distal ends 12a, 12b thereof expand radially outwardly in an arcuate fashion such that they are adapted to grasp the tissue and further secure the stent 12 to the wall of the passageway.

The stent 12 can be formed from a variety of materials that are not only biocompatible but also compressible and expandable to allow for the selective adjustment of the passageway. Such materials include woven fabrics or metals, and in an exemplary embodiment, the stent 12 can be formed of a woven wire mesh. An exemplary wire mesh material can be adapted to have a predetermined length and diameter in a neutral condition. A force, such as tension, can be applied to the device to cause it to reconfigure in an altered condition, such as a condition having an increased length and a reduced diameter. When the shape-altering force is removed from the device, it returns to its neutral condition. Examples of such material include shape memory metals such as NITINOL.

While the wire mesh stent can be formed in variety of ways, in an exemplary embodiment the stent can be formed from a continuously woven wire tube. The wires of the tube can be tac welded at a series of circumferential wire junctions to form the wire mesh, and then cut using any technique that will leave a series of exposed wire ends. Exemplary cutting techniques will be apparent to one having skill in the art and can include laser cutting techniques. The cut wire ends can then be shape set so to be disposed radially outwardly to effect fastening of the stent to the tissue upon activation of the stent, as noted above. One skilled in the art will appreciate the variety of other techniques that can be used to form a compressible and expandable wire stent.

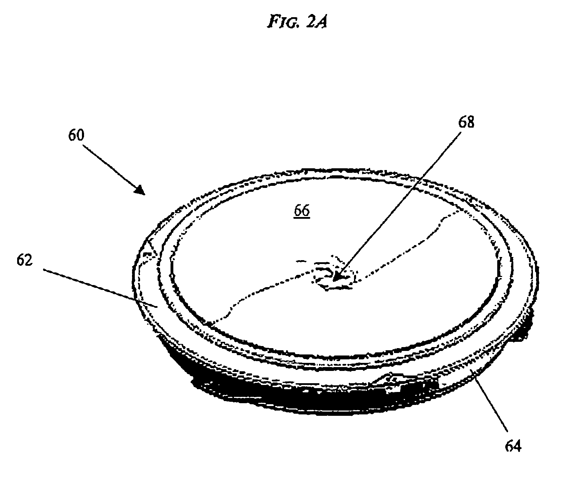

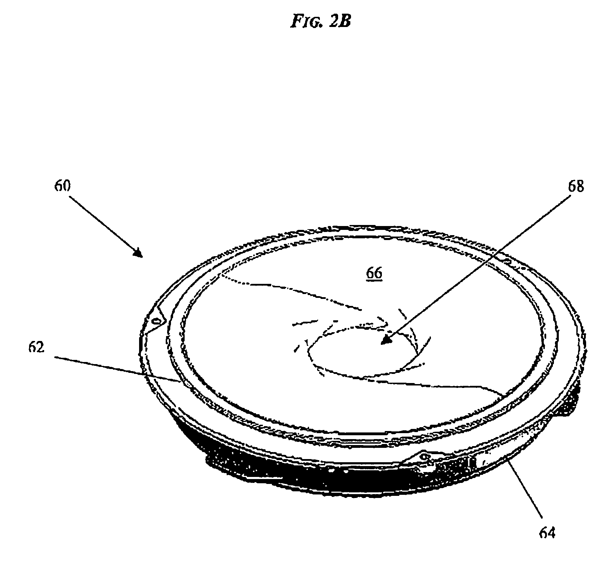

In another embodiment, as shown in FIGS. 2A-2B, the restriction element can be a cylindrical member 60 having upper and lower rings 62, 64 (which together form a housing) with a seal 66 that defines a central opening 68. As shown, the seal 66 extends radially inwardly from the upper and lower rings 62, 64 such that insertion and securement of the cylindrical member 60 within the passageway (e.g., the stomach) causes the passageway to decrease in diameter. The opening 68 in the seal 66 can have a selectively controllable diameter, and the upper ring 62 can be adapted to rotate relative to the lower ring 64 to control the size of the opening 68 so that the cylindrical member 60 can achieve a variety of sizes depending upon the type of passageway to be controlled and the needs of a patient.

The device 60 has a generally circular shape in its neutral (i.e., activated) position as shown in FIGS. 2A-2B. The upper and lower rings 62, 64 which form the housing of the device 60, and the seal 66, can all be pliable and deformable such that the device can be configured in an inactivated (i.e., delivery) position (not shown) in which the diameter of the device 60 is reduced and suitable for endoscopic (e.g., transoral) delivery. The inactivated position can be achieved by radially compressing the device 60, such as by configuring it to be placed within a delivery device (not shown), which, by way of example, may be a tubular member with an internal lumen. Upon release of the deforming forces, such as due to the removal of the delivery device, the device 60 returns to its neutral, activated position.

In one embodiment, the lower ring 64 can be adapted to anchor the device 60 to the wall of the passageway, and in an exemplary embodiment, the lower ring 64, and preferably the distal portion thereof, can have at least one attachment element (not shown) formed thereon that is adapted to receive a fastener such as a staple, tack, or suture, that couples the lower ring 64 to the wall of the gastrointestinal passageway. While the attachment element can have any configuration that allows it to receive a fastener, in an exemplary embodiment the attachment element can be a hook or a loop. In another embodiment, and optionally or in addition to the attachment element(s), the distal portion of the lower ring 64 can include a wire mesh band (also not shown) that is attached thereto. The band can have wire ends that are adapted to outwardly radially expand (e.g., upon release of a compressive force) to engage the wall of the passageway, similar to the wire ends 14 discussed above with respect to the stent 12 of FIGS. 1A-1B.

The upper ring 62 is axially secured to the lower ring 64 by a variety of mechanisms known in the art, however in an exemplary embodiment the seal 66 can be coupled between the upper and lower rings 62, 64 thereby joining them together. While the seal 66 can be coupled to the upper and lower rings 62, 64 in a variety of ways, in an exemplary embodiment it can be secured to an inner edge thereof by a fastener such as an adhesive or it can be secured with an o-ring.

The upper and lower rings 62, 64 are also adapted to rotate relative to one another such that the rotation controls configuration of the seal 66 to selectively adjust the diameter of the opening 68. While rotation can be effected by a variety of techniques, in an exemplary embodiment the upper and lower rings 62, 64 are biased to contact one another, and surface features present on the facing surfaces of the upper and lower rings 62, 64 prevent relative rotation of the rings unless the biasing force is overcome. In one embodiment, the upper and lower rings 62, 64 are biased into contact with each other and a pawl-like surface feature(s) on the facing surface of one ring engages a ratchet-like surface feature(s) on the facing surface of the other ring. The biasing of these surfaces toward each other prevents relative rotation of the rings until the biasing force is overcome so that the pawl and ratchet-like surface features can be disengaged. The ratchet and pawl-like surface features can be formed continuously around the facing surface of the upper and lower rings, or in a spaced relationship therearound, however in an exemplary embodiment the ratchet-like surface feature(s) and corresponding pawl-like surface feature(s) are formed continuously around the facing surface the upper and lower rings. One skilled in the art will appreciate that the rings can be joined in a number of other ways to enable selective, relative rotation of the rings to control a diameter of the opening 68.

In one embodiment, control of the opening diameter can be effected by relative rotation of the upper and lower rings 62, 64. By way of example, the opening 68 can be decreased by separating the rings and rotating the upper ring 62 relative to the lower ring 64 in a clockwise direction. Upon release of the tensioning (i.e., separating) forces, the facing surfaces of the rings will engage one another and the surface features will prevent further relative rotation. The opening size can be increased in a similar manner by relative counterclockwise rotation.

One skilled in the art will appreciate that the size of the opening need not be adjustable. Instead it can have a static, predetermined diameter, for example, in the range of about 1-10 mm. When the opening 68 has an adjustable diameter, the opening size can range from about 1-15mm. FIG. 2A illustrates the device 60 having an opening 68 with a smaller diameter than the opening 68 shown in FIG. 2B.

The cylindrical member 60 can be formed from a variety ofbiocompatible materials. For example, the upper and lower rings 62, 64 are preferably formed from a thermoplastic material that has sufficient pliability and flexibility that it can attain the inactivated position as discussed above, yet return to its neutral shape upon removal of a deforming force. Examples of suitable materials include Nylon 66, polyethylene, polypropylene, polyetheretherketone (PEEK), and polysulfone. Further, the seal 66 can be formed from an elastomeric material such as silicone, isoprene, combinations thereof, and other similar materials known in the art. The seal 66 can also be formed from a wire mesh, similar to the wire mesh used to form the stent 12 discussed above in FIGS. 1A-1B. The wire mesh seal can optionally be covered with a sheath (not shown) to direct food into the opening and prevent food from passing through the mesh.

One skilled in the art will appreciate that the cylindrical member 60 can have a variety sizes depending upon the type of passageway controlled. In an exemplary embodiment, where the passageway is the gastrointestinal passageway, and the device is intended to reduce the stomach volume, once the cylindrical member 60 is positioned at the desired tissue site, the seal 66 can be reconfigured from the inactivated position to the activated position as described above to reduce the size of the stomach. The overall diameter of the cylindrical member 60 can be the same in both the inactivated position and the activated position, and in an exemplary embodiment, the diameter of the device is in the range of about 1-4 cm.

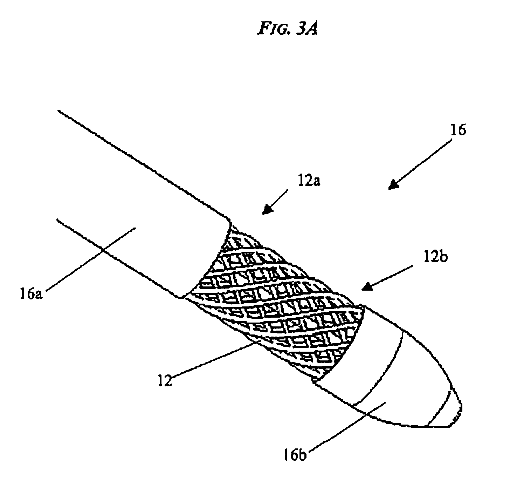

Methods for suppressing appetite by controlling the size of a gastrointestinal passageway are also disclosed herein. In one exemplary method, a cylindrical member, serving as a restriction element such as either of those shown in FIGS. 1A-2B, can be inserted into a site within the gastrointestinal passageway in an inactivated position. By way of non-limiting example, as shown in FIGS. 3A-3D, the restriction element is inserted endoscopically, e.g., transorally and through the esophagus into the proximal portion of the stomach 21a, to reduce the size of the stomach. Such reduction in the size of the stomach reduces food intake by contributing a sense of satiety with less food intake, thus leading to weight loss.

A variety of devices can be used to insert the restriction element into the passageway, and exemplary delivery devices include flexible, tubular elements that are adapted to be inserted into a passageway endoscopically, and in particular, transorally. For example, an exemplary delivery device can have an outside diameter that is less than the internal diameter of a lumen through which the device is to be inserted. The device should also be slightly flexible such that it can adapt to the shape of the lumen and maneuver therethrough. The delivery device should also be configured such that the restriction element can be easily disengaged therefrom. Exemplary delivery devices include a guide wire, an endoscopic grasper, a tube within an internal lumen, or any other delivery device known in the art can be used to insert the restriction element into the passageway.

For example and referring back to FIG. 3A, when the restriction element is a stent 12 such as that shown in FIGS. 1 A-1B, the delivery element 16 can be an elongate tubular element having proximal and distal sheaths 16a, 16b. The proximal sheath 16a is adapted to receive the proximal end 12a of the stent 12 and the distal sheath 16b is adapted to receive the distal end 12b of the stent 12. Once the ends of the stent 12a, 12b are engaged within the sheaths 16a, 16b, the sheaths 16a, 16b hold the ends 12a, 12b in a tensioned, inactivated position and prevent it from expanding prior to insertion into the passageway. In other embodiments, when the restriction element is a cylindrical housing, such as cylindrical member 60 discussed in FIGS. 2A-2B, a guide wire, a grasper, or a tubular device can be used to facilitate insertion into the passageway.

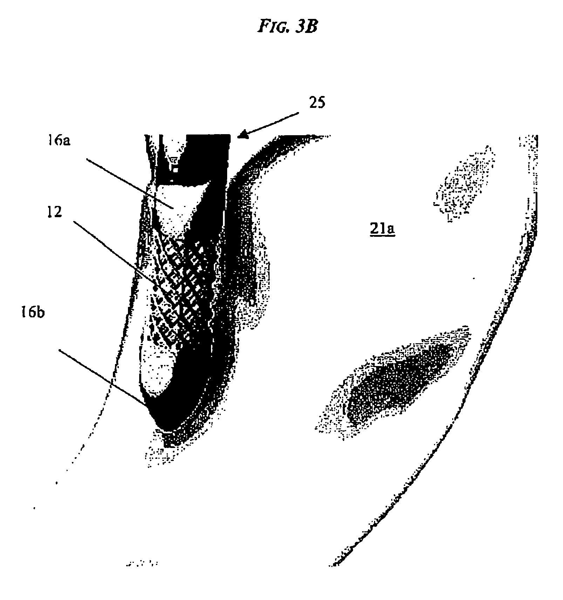

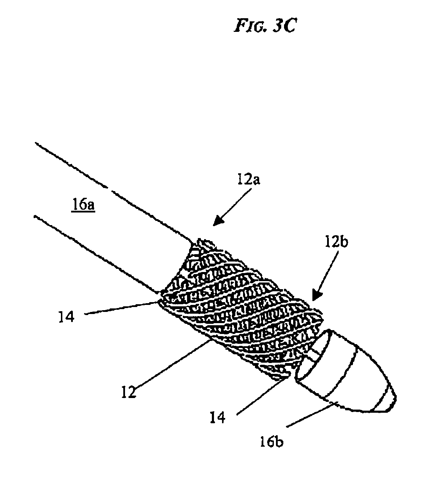

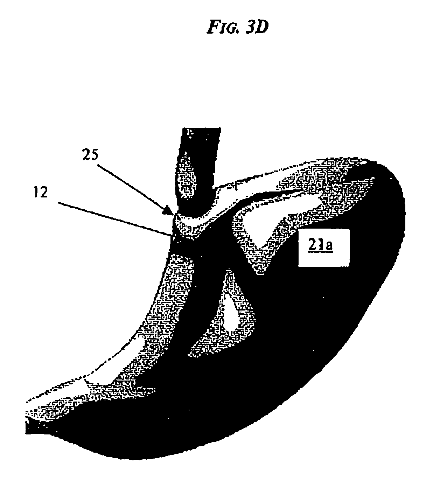

Once the restriction element is inserted at the target site, the restriction element can be coupled to at least a portion of the wall of the gastrointestinal passageway in a variety of ways. This can occur substantially simultaneously with or prior to activation of the restriction element. In one embodiment, when a stent is used, such as stent 12 of FIGS. 1A-1B, coupling of the restriction element occurs substantially simultaneously with activation of the restriction element. In particular, and as shown in FIGS. 3B-3C, suction can be applied through the delivery device 16 to pull the walls of the passageway 25 to the stent 12, such that the walls are engaged by the wire mesh thereof. As a result, a majority of the circumference of the stent 12 (e.g., about 350 degrees) is surrounded with the walls of the passageway 25, and the diameter of the passageway 25 is thus reduced. While suction is applied, the stent 12 can be released from the delivery device 16 and into the activated position, such that the stent radially expands and axially contracts. In particular, the proximal and distal ends 12a, 12b of the stent 12 can be released from the proximal and distal sheaths 16a, 16b of the delivery device 16. As a result of the radial expansion of the stent 12, the wire ends 14 also radially expand in an arcuate fashion and into the wall of the gastrointestinal passageway to further pull the wall of the passageway towards the stent 12 and ensure contact therewith. Once the activated, the application of suction can cease and the delivery device can be removed from the passageway. As a result, and as shown in FIG. 3D, for example, the restriction element 12 is left within the passageway 25, such that the passageway 25 has a new, and preferably restricted, diameter, which also causes a reduction in the stomach volume.

Activation of the device can also occur after the device is coupled to the wall of the passageway. For example, where a cylindrical housing, such as cylindrical member 60 of FIGS. 2A-2B, is used, the device can be secured to the wall of the passageway by coupling a fastener to an attachment element formed on the lower ring. Alternatively or additionally, as noted above the distal portion of the lower ring can include a band of wire mesh having expandable wire ends, similar to that as discussed above with respect to the stent. As a result, and once the device is coupled to the wall of the passageway, the wall of the passageway is decreased due to the inwardly extending seal. The opening size can be adjusted, if desired, by rotating the upper ring in the manner described above.

One skilled in the art will further appreciate that a variety of other techniques can be used with the methods disclosed herein to ensure formation of the new passageway. In one embodiment, for example, axially-extending staples can be applied alongside the inserted restriction element on the outer surface of the passageway to ensure maintenance thereof.

One skilled in the art will appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.

## Claims

1. A device for controlling the size of a gastrointestinal passageway, comprising:
a cylindrical member having proximal and distal ends, at least one of the proximal and distal ends being adapted to couple to a wall of the gastrointestinal passageway such that the size of the passageway is decreased, wherein the member is selectively configurable in a first, inactivated position and a second, activated position, such that a diameter of the member is larger in the activated position than in the inactivated position.

2. The device of claim 1, wherein the cylindrical member is a wire mesh stent having proximal and distal ends with plurality of circumferentially disposed wire ends, wherein the wire ends are adapted to be disposed radially outwardly in the activated position.

3. The device of claim 2, wherein the cylindrical member, when in the activated position, has a radially expanded diameter and an axially contracted length.

4. The device of claim 2, wherein the diameter of the stent increases about 2.5 times when the stent is moved from the inactivated position to the activated position.

5. The device of claim 2, wherein the diameter of the stent in the inactivated position is in the range of about 5-15 mm and the diameter of the stent in the activated position is in the range of about 20-30 mm. ,

6. The device of claim 2, wherein the diameter of the stent in the inactivated position is about 10 mm and the diameter of the stent in the activated position is about 25 mm.

7. The device of claim 1, wherein the cylindrical member has an upper and lower ring with a seal extending inwardly from the upper and lower ring, the seal defining an opening having a diameter.

8. The device of claim 7, wherein the diameter of opening can be selectively controllable by relative rotation of the upper and lower rings.

9. The device of claim 7, wherein the seal is made from a material selected from the group consisting of silicone, isoprene, wire mesh, and combinations thereof.

10. The device of claim 7, wherein the seal is adapted to open to form an opening having a diameter in the range of about 1-15 mm.

11. The device of claim 7, wherein a distal portion of an outer surface of the lower ring includes a wire mesh band that is attached thereto and adapted to couple to the wall of the gastrointestinal passageway.

12. The device of claim 7, wherein the lower ring includes an attachment element that is adapted to receive a fastener that couples the lower ring to the wall of the gastrointestinal passageway.

13. A system for controlling the size of a gastrointestinal passageway, comprising:
a tubular restriction element adapted to couple to the walls of the gastrointestinal passageway such that the size of the passageway is decreased, wherein the restriction element has a diameter that is adjustable when the restriction element is moved from an inactivated position to an activated position; and
an activation device adapted to manipulate the restriction element such that it moves from the inactivated position to the activated position.

14. The system of claim 13, wherein the restriction element is a wire mesh stent adapted to radially expand and axially contract when moved from the inactivated position to the activated position.

15. The system of claim 14, wherein the restriction element includes proximal and distal ends with plurality of circumferentially disposed wire ends formed thereon that are adapted to engage tissue upon movement of the stent from the inactivated position to the activated position.

16. The system of claim 14, wherein the activation device is a delivery device that includes proximal and distal portions that are respectively adapted to engage the proximal and distal ends of the stent and to selectively release the proximal and distal ends of the stent to enable the stent to move from the inactivated position to the activated position.

17. The system of claim 13, wherein the restriction element is an adjustable housing having an upper and lower ring with a seal extending inwardly from the upper and lower ring, the seal defining an opening having a diameter and the diameter of the opening can be selectively controllable by relative rotation of the upper and lower rings.
